(19) Europäisches Patentamt
European Patent Office
Office européen des brevets

(11) Veröffentlichungsnummer: **0 100 981**
**B1**

# (12) EUROPÄISCHE PATENTSCHRIFT

(45) Veröffentlichungstag der Patentschrift: **18.01.89**

(51) Int. Cl.⁴: **A 61 L 15/04**, A 61 L 17/00, A 61 F 2/00 // C08L67/04

(21) Anmeldenummer: **83107529.6**

(22) Anmeldetag: **30.07.83**

## (54) Resorbierbare Knochenwachse.

(30) Priorität: **07.08.82 DE 3229540**

(43) Veröffentlichungstag der Anmeldung: **22.02.84 Patentblatt 84/8**

(45) Bekanntmachung des Hinweises auf die Patenterteilung: **18.01.89 Patentblatt 89/3**

(84) Benannte Vertragsstaaten: **AT BE CH DE FR GB IT LI LU NL SE**

(56) Entgegenhaltungen:
**EP-A- 0 085 944**
**EP-A- 0 086 401**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Ritter, Wolfgang, Dr., Hochdahlerstrasse 22, D-4010 Hilden (DE)**

EP 0 100 981 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Zur mechanischen Blutstillung an körpereigenem Hartgewebe beispielsweise am Knochen, ist es üblich, resezierte Knochenteile mit Knochenwachs zu behandeln. Tafeln aus Knochenwachs werden mit der gleichen Zielsetzung auch zur Abdeckung von mit Spongiosa ausgefüllten Räumen eingesetzt.

Die bisher verwendeten wachsartigen Massen sind beispielsweise aus Bienenwachs, Mandelöl und Salicilsäure oder Bienenwachs und Isopropylpalmitat aufgebaut. Zur einschlägigen Literatur sei beispielsweise verwiesen auf

B.L. Douglas, Oral. Sug., 6, 1195 (1953)

H.S. Selden, Oral. Surg. 29, 262 (1970)

T.W. Shields, General Thoracic Surgery, Lea and Febiger, Philadelphia (1972)

D. Wolter, et al, Chirurg. 46, 459 (1975)

Die postoperative Heilung verläuft im allgemeinen ungestört, bakterielle Kontaminationen sind unüblich.

Generell wird allerdings bei den bisher beispielsweise zur Blutstillung bei Operationen eingesetzten Knochenwachsen eine Einscheidung des Implantats durch Granulationsgewebe beobachtet, in welchem sich reichlich Makrophagen und Riesenzellen befinden, vergleiche D. Wolter, et al, a.a.o. Das Granulationsgewebe wird im Körper im Laufe der Zeit fibrosiert. Ein direkter Kontakt zwischen Knochen und Wachs kommt nicht zustande. An den Kontaktzonen Spongiosa/Knochenwachs treten häufig unspezifische Fremdkörperreaktionen auf. Hierdurch wird die Knochenneubildung gehemmt und die Entstehung von Pseudoarthrosen begünstigt, siehe hierzu

I.R. Geary, et al, Ann. Surg. 132, 1128 (1950) und

C.C. Howard, et al, Clin. Orthop. 63, 226 (1969).

Die vorliegende Erfindung geht von der Aufgabe aus, auf dem hier betroffenen Gebiet der Knochenwachse Massen anzubieten, die aufgrund ihrer Konsistenz die bisher geforderten Aufgaben eines Knochenwachses übernehmen können, gleichzeitig aber – veranlasst durch ihren chemischen Aufbau – die Nachteile der bisherigen Knochenwachse vermeiden. Die erfindungsgemässen wachsartigen Massen sollen insbesondere physiologisch unbedenklich und gut resorbierbar sein, ohne dabei zur Bildung toxischer Abbauprodukte zu führen. Die Erfindung will in einer besonderen Ausgestaltung eine steuerbare Abbaugeschwindigkeit dieser wachsartigen Massen durch körpereigene Abbaureaktionen ermöglichen, so dass unspezifische Fremdkörperreaktionen und insbesondere chronische Entzündungen an den Kontaktzonen-Gewebe/Knochenwachs vermieden werden können. Durch die Resorbierbarkeit der erfindungsgemässen Massen soll weiterhin Einfluss auf eine ungehemmte Knochenneubildung genommen werden.

Gegenstand der Erfindung sind dementsprechend in einer ersten Ausführungsform resorbierbare Wachse zur mechanischen Blutstillung an körpereigenem Hartgewebe, insbesondere Knochen, wobei diese Wachse dadurch gekennzeichnet sind, dass sie aus bei Körpertemperatur zähviskosen bis festen wachsartigen Polyester-Oligomeren eines mittleren Molekulargewichts von 200–1500 von Hydroxycarbonsäuren mit 2–10 C-Atomen bestehen. In der bevorzugten Ausführungsform sind diese Polyester-Oligomere aus Monohydroxy-Monocarbonsäuren gebildet, wobei es allerdings möglich ist, dass an einem entsprechenden Grundkörper solcher Polyester-Oligomere bevorzugt endständig Coreaktanten angebunden sind.

Die Oligomersegmente dieser bevorzugten Wachsmassen weisen das Strukturmerkmal

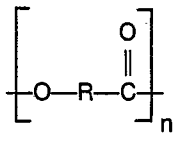

auf. Sie sind durch Oligomerisierung geeigneter Hydroxycarbonsäuren beziehungsweise Hydroxycarbonsäuregemische

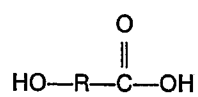

zugänglich. In der Erfindung sind die Reste –R– und n derart ausgewählt beziehungsweise aufeinander abgestimmt, dass das mittlere Molekulargewicht der Polyester-Oligomereinheit im Bereich von 200 bis 1500 liegt. Besonders bevorzugte Werte für das mittlere Molekulargewicht liegen im Bereich von 300 bis 1000. Die für die Anwendung gewünschte Konsistenz wird durch gezielte Einstellung des Oligomerisierungsgrades erreicht.

Das Polyester-Oligomersegment wird in einer bevorzugten Ausführungsform der Erfindung aus Monohydroxy-Monocarbonsäuren gebildet, die eine C-Zahl von 10 im Molekül nicht überschreiten.

Polyester-Oligomere aus entsprechenden Säuren aus 2 bis 10, insbesondere 2 bis 6 Kohlenstoffatomen im Molekül können besonders wichtig sein. Es können dabei zur Ausbildung des Polyester-Oligomeren bestimmte ausgewählte einzelne Hydroxycarbonsäuren oder aber auch Gemische verschiedener Hydroxycarbonsäuren zur Verwendung kommen.

Besonders wichtige Hydroxycarbonsäuren sind in diesem Zusammenhang Glycolsäure, die isomeren Milchsäuren, die gegebenenfalls isomeren α- oder β-Hydroxypropionsäuren, die gegebenenfalls isomeren α-, β- oder γ-Hydroxybuttersäuren, o-Hydroxybenzoesäure (Salicylsäure), m-Hydroxybenzoesäure und/oder p-Hydroxybenzoesäure (Anissäure). Es können dabei bestimmte Isomere der genannten Säuren als auch Gemische zum Einsatz kommen.

In einer besonders bevorzugten Ausführungsform ist das Polyester-Oligomere im wesentlichen aus Glycolsäure und/oder den isomeren Milchsäuren ausgebildet. Durch gemeinsame Verwendung dieser Reaktanten lässt sich weitgehend Einfluss auf die Abbaugeschwindigkeit des Wachses durch körpereigene Abbaureaktionen nehmen.

Hochpolymere der hier geschilderten Art und ihr Einsatz auf dem medizinischen Sektor sind bekannt. Sie besitzen Fasereigenschaften. Ihre Verträglichkeit und Abbaubarkeit sind eingehend untersucht. Bekannt sind beispielsweise synthetische im Organismus resorbierbare Fadenmaterialien auf der Basis von Polyglycolsäure und Polymilchsäure, vergleiche hierzu beispielsweise die US-PSen 3 297 033, 3 422 871, 3 626 948, 2 668 162, 2 676 945 und 2 703 316.

Hochpolymere Materialien dieser Art haben sich beispielsweise für Anwendungen in der Zahnmedizin, der Orthopädie und zum gezielten Freisetzen von Pharmaca durchgesetzt, sie sind vergleichbaren natürlichen Produkten, zum Beispiel Catgut deutlich überlegen. Auch zur Abbaubarkeit dieser Polymeren liegt umfangreiches Datenmaterial vor, vergleiche hierzu insbesondere
R.A. Miller, et al, J. Biomed., Mat. Res. 11, 711–719 (1977)
D.C. Miln, et al, Scot. med., J. 17, 108 (1972)
A.M. Reed, et al, Polymer 22, 494 (1981).

Dabei kann in an sich bekannter Weise mit Hilfe von Cokondensaten aus Glycolsäure und Milchsäure die Abbaugeschwindigkeit innerhalb weiter Grenzen wunschgemäss eingestellt werden – vergleiche hierzu die zitierte Veröffentlichung R.A. Miller, et al, J. Biomed., Mat. Res. 11, 711–719 (1977). Dieses grundsätzliche Wissen kann im Rahmen der Erfindung für die Zusammensetzung der erfindungsgemäss jetzt vorgeschlagenen wachsartigen Polyester-Oligomeren mit verwendet werden.

Polyester-Oligomere aus Hydroxycarbonsäuren können zwar unmittelbar durch Polykondensation der Hydroxycarbonsäuren beziehungsweise Hydroxycarbonsäuregemische hergestellt werden, zur gezielten Einstellung des Oligomerisierungsgrades – und damit der gewünschten wachsartigen zähviskosen bis festen Konsistenz des Reaktionsproduktes ist es jedoch sinnvoll, in bekannter Weise die Einstellung der gewünschten Oligomerisierungsgrade zu regeln. In diesem Sinne ist es bevorzugt bei der Herstellung der Polyester-Oligomeren das Molekulargewicht regelnde Coreaktanten mitzuverwenden wobei hier in erster Linie Alkohole, Carbonsäuren und/oder Amine in Betracht kommen können.

Als Coreaktanten sind in erster Linie monofunktionelle Alkohole, monofunktionelle Carbonsäuren oder monofunktionelle Amine geeignet, es können aber auch mehrfunktionelle, insbesondere difunktionelle Alkohole oder Carbonsäuren mitverwendet werden. In allen Fällen gelingt in an sich bekannter Weise die Regelung des mittleren Molekulargewichts im Polyester-Oligomeren und damit die Einstellung der angestrebten Viskositätsbereiche für die erfindungsgemässen Knochenwachse.

In der Auswahl der in der Regel nur in untergeordneten Mengen mitverwendeten monofunktionellen oder difunktionellen Coreaktanten sind praktisch keine Einschränkungen gegeben. So können diese Reaktanten aliphatischer, zykloaliphatischer oder aromatischer Natur sein. Sie besitzen eine Kohlenstoffzahl nicht über $C_{25}$, vorzugsweise nicht über $C_{15}$. Geeignete Diole enthalten beispielsweise 2–20 C-Atome, vorzugsweise 2–10 und insbesondere 2–6 C-Atome im Molekül. Die gleichen Angaben gelten für die entsprechenden Dicarbonsäuren. Die hier genannten Zahlenwerte haben ebenso Gültigkeit für entsprechende monofunktionelle Alkohole beziehungsweise Carbonsäuren.

Beispiele für geeignete Diole sind:
Ethylenglycol, Di-, Tri-, Tetraethylenglycol,
Propylenglycol, Dipropylenglycol,
1,3-Butylenglycol, 1,4-Butylenglycol,
2,2-Dimethyl-1,3-propandiol,
2,2,4-Trimethyl-1,6-hexandiol,
1,4-Cyclohexandimethanol, 1,5-Pentandiol,
1,6-Hexandiol, 1,10-Decandiol und
2,2-Bis-(4-hydroxycyclohexyl)-propan.

Geeignete Dicarbonsäuren sind beispielsweise:
Oxalsäure, Malonsäure, Bernsteinsäure,
Glutarsäure, Adipinsäure, Pimelinsäure,
Korksäure, Azelainsäure, Sebazinsäure,
Isosebazinsäure, Nonandicarbonsäure,
Decandicarbonsäure, Undecandicarbonsäure,
Dodecandicarbonsäure, Phthalsäure,
Hexahydrophthalsäure, Isophthalsäure,
Terephthalsäure und Biphenyldicarbonsäure.

Als monovalente Coreaktanten kommen entsprechende aliphatische, zykloaliphatische oder aromatische Monoalkohole, entsprechende Monocarbonsäuren und gegebenenfalls entsprechende Amine – hier insbesondere primäre oder auch sekundäre Amine – in Betracht.

Selbstverständlich können in allen Fällen – das heisst sowohl bei den Hydroxycarbonsäuren als auch bei den Coreaktanten – nicht nur die jeweils freien reaktiven Komponenten der genannten Art, sondern auch solche reaktive Derivate eingesetzt werden, die in an sich bekannter Weise unter den Bedingungen der Veresterung beziehungsweise Umesterung die gewünschten Polyester-Oligomeren des vorbestimmten Molekulargewichts bilden. Geeignet sind also beispielsweise die Ester der Hydroxycarbonsäuren, sowie Lactone beziehungsweise Lactame von Hydroxycarbonsäuren, die mit Diolen beziehungsweise Diolestern umgesetzt, insbesondere umgeestert werden können. Die Herstellung der Polyester-Oligomeren erfolgt in an sich bekannter Weise zum Beispiel durch Reaktion in Gegenwart oder in Abwesenheit von Lösungsmitteln, gewünschtenfalls in Gegenwart von Katalysatoren, insbesondere Veresterungskatalysatoren.

Werden Oligomere der geschilderten Art durch Cokondensation von Hydroxycarbonsäuren und Diolen hergestellt, so entstehen letztlich Polyester-Oligomeren mit endständigen Hydroxylgruppen. Die Mengen der mitverwendeten Diole bestimmt – in Abstimmung mit den Reaktionsbedingungen – das mittlere Molekulargewicht des anfallenden Polyester-Oligomeren.

Werden andererseits die Oligomeren durch Cokondensation von Hydroxycarbonsäuren mit Dicarbonsäuren beziehungsweise reaktiven Dicarbonsäurenderivaten hergestellt, so entstehen Polyester-Oligomere mit endständigen Carboxylgruppen beziehungsweise Carboxylgruppenderivate. Auch hier wirkt der mitverwendete Coreaktant gleichzeitig vereinheitlichend bezüglich der endständigen reaktiven Gruppen und molekulargewichtsregelnd. Im einzelnen gilt hier das bekannte Wissen zur Herstellung von Polyester beziehungsweise Copolyestern. Die Mitverwendung von Monoalkoholen und/oder Monoaminen führt zur bevorzugten Blockierung der endständigen Carbonsäuregruppierung im Polyester-Oligomeren, die Mitverwendung von Monocarbonsäuren blockiert dementsprechend die endständige Hydroxylgruppenseite des Oligomeren.

In der bevorzugten Ausführungsform der Erfindung sind die hier beschriebenen Knochenwachse bei Körpertemperatur – d. h. im Temperaturbereich von 35–40 °C fest. Ihre mechanische Beschaffenheit kann dabei so ausgewählt werden, dass sie innerhalb dieses Temperaturbereichs pastös beziehungsweise weich verstreichbar sind, um dem Anwendungszweck optimal zu genügen. Geeignet sind allerdings insbesondere auch solche Polyester-Oligomere, die im Bereich der Körpertemperatur relativ fest und hart sind und durch mässiges Erwärmen in einen pastös bis weich verstreichbaren Zustand gebracht werden können.

In jedem Einzelfall sind bei der praktischen Anwendung stets ja nur sehr kleine Portionen des Knochenwachses zum Verschluss eines Blutgefässes einzusetzen. Diese Portionen können beispielsweise auf einem Spatel durch geringfügiges Vorwärmen in den gewünschten pastösen Zustand gebracht und in dieser Form angewendet werden woraufhin sie im Bereich der Körpertemperatur zum festen Verschlussmaterial erhärten. Geeignet sind beispielsweise entsprechende Wachse die bei Temperaturen bis 100 °C vorzugsweise bis 60 °C den gewünschten Grad an weicher Verarbeitungsfähigkeit entwickeln.

Beispiele

Beispiele 1–3

In einem Dreihalskolben mit Rührer und Destillationsbrücke werden Glycolsäure und Ethylenglycol vorgelegt. Unter Stickstoff wird schnell auf 150 °C und dann im Verlauf von 6 Stunden von 150 auf 200 °C hochgeheizt. Dabei spaltet sich bereits der grösste Anteil des Reaktionswassers, der den Umsatz der Esterkondensation anzeigt, ab. Man lässt den Ansatz auf etwa 150 °C abkühlen, evakuiert vorsichtig auf 10 Torr und vervollständigt den Umsatz bei 200 °C und 10 Torr. Nach 30 Minuten wird das Produkt bei ca. 150 °C heiss abgefüllt. Die Zusammensetzung der Ansätze und die Oligomereigenschaften sind der Tabelle 1 (Beispiel 1–3) zu entnehmen.

Tabelle 1
Oligohydroxycarbonsäuren aus Glycolsäure und Ethylenglycol

| Beispiel | Edukte Glycolsäure mol | Ethylenglycol mol | Ausbeute Reaktionswasser/% | Beschaffenheit |
|---|---|---|---|---|
| 1 | 3 | 1 | 100 | klar, viskos, hellgelb |
| 2 | 4 | 1 | 90 | viskos, weiss |
| 3 | 6 | 1 | 98 | wachsartig, weiss |

Beispiel 4–8

In einem Dreihalskolben mit Rührer und Destillationsbrücke werden Dicarbonsäure und Hydroxycarbonsäure vorgelegt. Unter Stickstoff wird schnell auf 150 °C und dann im Verlauf von 6 Stunden von 150 auf 200 °C hochgeheizt. Dabei spaltet sich bereits der grösste Teil des Reaktionswassers, der den Umsatz der Esterkondensation anzeigt, ab. Man lässt den Ansatz auf etwa 150 °C abkühlen, evakuiert vorsichtigt auf 10 Torr und vervollständigt den Umsatz bei 200 °C und 10 Torr. Das Produkt wird heiss unter Stickstoff abgefüllt. Die Zusammensetzung der Ansätze und die Oligomereigenschaften sind in der Tabelle 2 (Beispiel 4 bis 8) zu entnehmen.

Tabelle 2
Oligohydroxycarbonsäuren aus Glycolsäure und Adipinsäure

| Beispiel | Edukte Glycolsäure mol | Adipinsäure mol | Ausbeute Reaktionswasser/% | Beschaffenheit |
|---|---|---|---|---|
| 4 | 1 | 1 | 99 | wachsartig, fest |
| 5 | 2 | 1 | 99 | wachsartig |
| 6 | 3 | 1 | 98 | wachsartig, weich |
| 7 | 4 | 1 | 96 | wachsartig, weich |
| 8 | 6 | 1 | 98 | wachsartig, weich |

**Patentansprüche für die Vertragsstaaten BE, CH, DE, FR, GB, IT, LI, LU, NL, SE**

1. Resorbierbare Wachse zur mechanischen Blutstillung an körpereigenem Hartgewebe, insbesondere Knochen, dadurch gekennzeichnet, dass sie aus bei Körpertemperatur zähviskosen bis festen wachsartigen Polyester-Oligomeren eines mittleren Molekulargewichtes von 200 bis 1500 von Hydroxycarbonsäuren mit 2 bis 10 C-Atomen bestehen.

2. Resorbierbare Wachse nach Anspruch 1, dadurch gekennzeichnet, dass das Polyester-Oligomere ein mittleres Molekulargewicht im Bereich von 300 bis 1000 besitzt.

3. Resorbierbare Wachse nach Anspruch 1 bis 2, dadurch gekennzeichnet, dass das Polyester-Oligomere aus Monohydroxycarbonsäuren gebildet ist, die 2 bis 6 C-Atome aufweisen.

4. Resorbierbare Wachse nach Anspruch 1 bis 3, dadurch gekennzeichnet, dass das Polyester-Oligomere aus Glycolsäure, Milchsäure, Hydroxypropionsäure, Hydroxybuttersäure und/oder Hydroxybenzoesäure aufgebaut ist.

5. Resorbierbare Wachse nach Anspruch 1 bis 4, dadurch gekennzeichnet, dass die Polyester-Oligomere unter Mitverwendung von monofunktionellen und/oder difunktionellen Alkoholen oder Carbonsäuren bzw. Carbonsäureanhydriden und/oder unter Mitverwendung von primären und/oder sekundären Monoaminen mit jeweils nicht über 25 C-Atomen hergestellt worden sind.

6. Resorbierbare Wachse nach Anspruch 1 bis 5, dadurch gekennzeichnet, dass sie bei Körpertemperatur pastös bis weich verstreichbar sind oder durch kurzzeitiges Erwärmen auf Temperaturen bis 100 °C, vorzugsweise bis 60 °C, in einen pastös verstreichbaren Zustand gebracht werden können.

7. Verwendung der resorbierbaren Wachse nach Ansprüchen 1 bis 6 bei der mechanischen Blutstillung an körpereigenem Hartgewebe, insbesondere an Knochen.

**Patentansprüche für den Vertragsstaat AT**

1. Verfahren zur Herstellung resorbierbarer Wachse zur mechanischen Blutstillung an körpereigenem Hartgewebe, insbesondere Knochen, dadurch gekennzeichnet, dass sie als bei Körpertemperatur zähviskose bis feste wachsartige Polyester-Oligomere eines mittleren Molekulargewichtes von 200 bis 1500 von Hydroxycarbonsäuren mit 2 bis 10 C-Atomen ausgebildet werden.

2. Verfahren nach Anspruch 1, dadurch gekennzeichnet, dass das Polyester-Oligomer auf ein mittleres Molekulargewicht im Bereich von 300 bis 1000 eingestellt wird.

3. Verfahren nach Anspruch 1 bis 2, dadurch gekennzeichnet, dass das Polyester-Oligomere aus Monohydroxycarbonsäuren gebildet ist, die 2 bis 6 C-Atome aufweisen.

4. Verfahren nach Anspruch 1 bis 3, dadurch gekennzeichnet, dass das Polyester-Oligomere aus Glycolsäure, Milchsäure, Hydroxypropionsäure, Hydroxybuttersäure und/oder Hydroxybenzoesäure aufgebaut wird.

5. Verfahren nach Anspruch 1 bis 4, dadurch gekennzeichnet, dass die Polyester-Oligomere unter Mitverwendung von monofunktionellen und/oder difunktionellen Alkoholen oder Carbonsäuren bzw. Carbonsäureanhydriden und/oder unter Mitverwendung von primären und/oder sekundären Monoaminen mit jeweils nicht über 25 C-Atomen hergestellt werden.

6. Verfahren nach Anspruch 1 bis 5, dadurch gekennzeichnet, dass die resorbierbaren Wachse bei Körpertemperatur pastös bis weich verstreichbar sind oder durch kurzzeitiges Erwärmen auf Temperaturen bis 100 °C vorzugsweise bis 60 °C in einen pastös verstreichbaren Zustand gebracht werden.

7. Verwendung der resorbierbaren Wachse nach Ansprüchen 1 bis 6 bei der mechanischen Blutstillung an körpereigenem Hartgewebe, insbesondere an Knochen.

**Claims for the contracting states BE, CH, DE, FR, GB, IT, LI, LU, NL, SE**

1. Resorbable waxes for the mechanical staunching of blood on hard body tissue, more especially bones, characterized in that they consist of wax-like polyester oligomers of $C_2$–$C_{10}$ hydroxycarboxylic acids which are viscous to solid at body temperature and have an average molecular weight of 200 to 1500.

2. Resorbable waxes as claimed in claim 1, characterized in that the polyester oligomer has an average molecular weight in the range from 300 to 1000.

3. Resorbable waxes as claimed in claims 1 and 2, characterized in that the polyester oligomer is formed of $C_2$–$C_6$ monohydroxycarboxylic acids.

4. Resorbable waxes as claimed in claims 1 to 3, characterized in that the polyester oligomer is synthesized from glycolic acid, lactic acid, hydroxypropionic acid, hydroxybutyric acid and/or hydroxybenzoic acid.

5. Resorbable waxes as claimed in claims 1 to 4, characterized in that the polyester oligomers have been prepared using monofunctional and/or difunctional alcohols or carboxylic acids or carboxylic anhydrides and/or using primary and/or secondary monoamines containing no more than 25 C atoms.

6. Resorbable waxes as claimed in claims 1 to 5, characterized in that they are paste-like to soft-spreading at body temperature or can be brought into a paste-like soft-spreading form by brief heating to temperatures of up to 100 °C and preferably to temperatures of up to 60 °C.

7. The use of the resorbable waxes claimed in claims 1 to 6 in the mechanical staunching of blood on hard body tissue, more especially bones.

**Claims for the contracting state AT**

1. A process for the production of resorbable waxes for the mechanical staunching of blood on hard body tissue, more especially bones, characterized in that they are formed as wax-like polyester oligomers of $C_2$–$C_{10}$ hydroxycarboxylic acids which are viscous to solid at body temperature and have an average molecular weight of 200 to 1500.

2. A process as claimed in claim 1, characterized in that the polyester oligomer is adjusted to an average molecular weight in the range from 300 to 1000.

3. A process as claimed in claims 1 and 2, characterized in that the polyester oligomer is formed of $C_2$–$C_6$ monohydroxycarboxylic acids.

4. A process as claimed in claims 1 to 3, characterized in that the polyester oligomer is synthesized from glycolic acid, lactic acid, hydroxypropionic acid, hydroxybutyric acid and/or hydroxybenzoic acid.

5. A process as claimed in claims 1 to 4, characterized in that the polyester oligomers are prepared using monofunctional and/or difunctional alcohols or carboxylic acids or carboxylic anhydrides and/or using primary and/or secondary monoamines containing no more than 25 C atoms.

6. A process as claimed in claims 1 to 5, characterized in that the waxes are paste-like to soft-spreading at body temperature or can be brought into a paste-like soft-spreading form by brief heating to temperatures of up to 100 °C and preferably to temperatures of up to 60 °C.

7. The use of the resorbable waxes according to claims 1 to 6 in the mechanical staunching of blood on hard body tissue, more especially bones.

**Revendications pour les états contractants BE, CH, DE, FR, GB, IT, LI, LU, NL, SE**

1. Cires assimilables pour l'hémostase mécanique sur des tissus compacts de l'organisme, en particulier sur des os, caractérisées en ce qu'elles se composent d'oligomères de polyester cireux visqueux à solides à la température du corps d'un poids moléculaire moyen de 200 à 1500 d'acides hydroxycarboxyliques avec 2 à 10 atomes de carbone.

2. Cires assimilables selon la revendication 1, caractérisées en ce que l'oligomère de polyester possède un poids moléculaire dans l'intervalle de 300 à 1000.

3. Cires assimilables selon les revendications 1 à 2, caractérisées en ce que l'oligomère de polyester est formé à base d'acides monohydrocarboxyliques avec 2 à 6 atomes de carbone.

4. Cires assimilables selon les revendications 1 à 3, caractérisées en ce que l'oligomère de polyester est formé à base d'acide glycolique, d'acide lactique, d'acide hydroxypropionique, d'acide hydroxybutyrique et/ou d'acide hydroxybenzoïque.

5. Cires assimilables selon les revendications 1 à 4, caractérisées en ce que les oligomères de polyester ont été fabriqués sous utilisation d'alcools ou d'acides carboxyliques ou d'anhydrides d'acides carboxyliques monofonctionnels et/ou difonctionnels et/ou sous utilisation de monoamines primaires et/ou secondaires dont le nombre d'atomes de carbone ne dépasse respectivement pas 25.

6. Cires assimilables selon les revendications 1 à 5, caractérisées en ce qu'elles sont d'une consistance pâteuse à molle épandable à la température du corps ou en ce qu'elles peuvent être portées à un état pâteux épandable en les chauffant brièvement à des températures jusqu'à 100 °C, de préférence jusqu'à 60 °C.

7. Utilisation des cires assimilables suivant les revendications 1 à 6 lors de l'hémostase mécanique sur des tissus compacts de l'organisme, en particulier sur des os.

**Revendications pour l'état contractant AT**

1. Procédé de préparation de cires assimilables pour l'hémostase mécanique sur des tissus compacts de l'organisme, en particulier sur des os, caractérisé en ce que les cires sont formées d'oligomères de polyester cireux visqueux à solides à la température du corps d'un poids moléculaire moyen de 200 à 1500 d'acides hydroxycarboxyliques avec 2 à 10 atomes de carbone.

2. Procédé selon la revendication 1, caractérisé en ce que l'oligomère de polyester est ajusté en vue d'un poids moléculaire dans l'intervalle de 300 à 1000.

3. Procédé selon les revendications 1 à 2, caractérisé en ce que l'oligomère de polyester est formé à base d'acides monohydrocarboxyliques avec 2 à 6 atomes de carbone.

4. Procédé selon les revendications 1 à 3, caractérisé en ce que l'oligomère de polyester est formé à base d'acide glycolique, d'acide lactique, d'acide hydroxypropionique, d'acide hydroxybutyrique et/ou d'acide hydroxybenzoïque.

5. Procédé selon les revendications 1 à 4, caractérisé en ce que les oligomères de polyester sont fabriqués sous utilisation d'alcools ou d'acides carboxyliques ou d'anhydrides d'acides carboxyliques monofonctionnels et/ou difonctionnels et/ou sous utilisation de monoamines primaires et/ou secondaires dont le nombre d'atomes de carbone ne dépasse respectivement pas 25.

6. Procédé selon les revendications 1 à 5, caractérisé en ce que les cires assimilables sont d'une consistance pâteuse à molle épandable à la température du corps ou en ce qu'elles sont portées à un état pâteux épandable en les chauffant brièvement à des températures jusqu'à 100 °C, de préférence jusqu'à 60 °C.

7. Utilisation des cires assimilables suivant les revendications 1 à 6 lors de l'hémostase mécanique sur des tissus compacts de l'organisme, en particulier sur des os.